# EUROPEAN PATENT APPLICATION

(11) **EP 0 691 348 A1**
(43) Date of publication of application: **10.01.1996**
(21) Application number: 95111293.7
(22) Date of filing: 19.04.1988
(51) Int. Cl.: C07K 14/235, A61K 39/10, C07K 16/12

(54) **Immunogenic compositions prepared from adenylate cyclase of B. Parapertussis**

(62) Divisional of application: 88400949.9
(71) Applicant: INSTITUT PASTEUR, F-75724 Paris Cédex 15 (FR); INSTITUT NATIONAL DE LA SANTE ET DE LA RECHERCHE MEDICALE (INSERM), F-75654 Paris Cédex 13 (FR)
(72) Inventor: Rocancourt, Murielle, F-92140 Clamart (FR); Brezin, Colette, F-75007 Paris (FR); Boucaud, Jean-Luc, F-69470 Cours-La-Ville (FR); Szatanik, Marek, F-78210 Saint Cyr L'Ecole (FR); Alonso, Jean-Michel, F-78180 Montigny le Bretonneux (FR)
(74) Representative: Almond-Martin, Carol

(57) **Abstract**

The invention concerns an immunogenic composition characterised in that it contains an adenylate cyclase (AC) preparation, obtainable from B. parapertussis, said adenylate cyclase preparation comprising an active adenylate cyclase having a molecular weight (Mr) of about 43 ± 4.3 - 45 ± 4.5 kDa as determined with respect to ovalbumin as standard, specifically recognized by specific polyclonal and monoclonal anti-B. pertussis AC antibodies, and capable of antigenically cross-reacting with the B. pertussis AC, and/or an inactive protein having a molecular weight (Mr) of about 43 ± 4.3 kDa, specifically recognized by polyclonal anti-AC antibodies raised against purified AC preparation and devoid of adenylate cyclase calmodulin-activable activity and of affinity for calmodulin.

## Description

The invention relates to a method for obtaining protective antigens against Bordetella infections and toxic processes.

It is well known that B.pertussis and B.parapertussis are both causative agents of whooping cough outbreaks.

Studies on the virulence factors-prominently involved in the pathogenesis of whooping cough have led to consider two major products of B.pertussis, the filamentous hemagglutinin (FHA) and the pertussis toxin (Ptx), as major determinants of pathogenesis and as efficient immunogens.

In a previous work (FR patent application 8615968 of November 10, 1986 in the names of Institut Pasteur and Inserm), the inventors have used antibodies raised against an other product synthesized by B.pertussis, the adenylate cyclase (AC), to demonstrate the role of this molecule as major toxin in the pulmonary cytopathic syndrome, as evidenced by the induction of an acute adematous hemorragic alveolitis (AEHA) in mice, and thus, as potential protective antigen.

The inventors have now investigated the role of an adenylate cylase produced and released by another Bordetella species and have investigated its role in the pathogenesis and immunity in an experimental infection of the mouse.

It is then an object of the invention, using a Bordetella species different from B.pertussis, to provide adenylate cyclase preparations useful as protective antigens against whooping cough.

The method of the invention for obtaining an active adenylate cyclase and/or a cross-reactive, otherwise inactive protein, is characterized by the use of B.parapertussis or variants thereof.

It has to be noted that the terms "active" or "inactive" as used in the specification refer to the adenylate cyclase enzymatic activity.

The active adenylate cyclase has a molecular weight of about 43 ⁺ 4.3 - 45 ⁺ 4.5 kDa (molecular weight range Mr as determined with respect to ovalbumin used as standard). It is specifically recognized by specific polyclonal and monoclonal anti-B.pertussis AC antibodies.

Said active adenylate cyclase is capable of antigenically cross-reacting with the B.pertussis AC.

Said adenylate cyclase which is a cytotoxin responsible for the local pulmonary lesions occuring in whooping cough is advantageously endowed with immunogenic properties.

The inactive protein is disclosed in EP patent application filed on the same day, in the name of applicants, relating to "Immunoprotective antigenic protein against pathogenic bacteria".

Said inactive protein is specifically recognized by polyclonal anti-AC antibodies raised against purified AC preparations, and is devoid of adenylate cyclase CaM-activable activity and of affinity for CaM. It is also capable of antigenically cross-reacting with the B.pertussis AC.

A complex associating the active adenylate cyclase with the inactive, cross-reactive protein, is thus obtainable from B.parapertussis or variants expressing proteins with cross-reactivity.

The active and the inactive forms may thus be isolated by usual purification technics.

Surprisingly, said adenylate cyclase preparations are obtainable from B.parapertussis while it was admitted that cross-immunity does not exist between B.pertussis and B.parapertussis infections.

This aspect of the invention is of great interest since B.parapertussis does not express, as it is well known, the Ptx, while said toxin is only expressed by B.pertussis.

Accordingly, the invention provides means for obtaining antigenic adenylate cyclase preparations devoid of the side-effects associated with Ptx, such as the risks of allergic encephalitis.

According to the method of the invention, antigenic AC preparations, free from Ptx, are obtainable from B.parapertussis hly⁺ clones. Said clones are selected either in vitro or recovered from lungs homogenate post challenge and are capable of inducing an acute edematous haemorragic alveolitis.

A specific hly⁺ clone advantageously expressing high amounts of active AC was recovered from lungs and consists of 63-2S clone deposited under N°I-749 at the Collection Nationale de Culture de Microorganismes (CNCM), on April 15, 1988.

The culture supernatants of 63-2S corresponding to standardized bacterial suspensions at 10⁹ CFU/ml have an AC enzymatic activity of about 20-25 units.

Hly⁺ clones having a lower AC spontaneous expression can be injected by the intranasal route to mice, and reisolated from lungs, in order to increase the AC activity.

The derived clones maintaining hly⁺, AC⁺ phenotype expression enter into the scope of the invention. For example, clone 63-2TCS (trypto-casein-soy) has been obtained from 63-2S strain, growing on a non-specific medium with respect to Bordetellae, with the same yield and maintaining hly⁺, AC⁺ expression.

A cross immunity between B.pertussis and B.parapertussis was evidenced by passive cross protection with anti-B.pertussis adenylate cyclase antibodies. The works carried out on adenylate cyclase preparations obtained according to the invention have shown that said molecules are protective antigens.

Accordingly said adenylate cyclase preparations are advantageously used according to the invention for making vaccine preparations, under the proviso that they do not give cross-reactions with the host adenylate cyclase. The adenylate cyclase preparations comprise both active and inactive proteins, or only one of said proteins.

Such vaccines which are thus elaborated from adenylate cyclase preparations obtained from B.parapertussis are molecular vaccines and are capable of preventing Bordetella infections and toxic effects in human and animals. The usual dosis and administration forms are advantageously used. The vaccines are preferably under the form of intranasal, oral or parenteral preparations.

In the vaccine compositions, the adenylate cyclase preparations may be associated with FHA in the same inoculum or not. FHA will then be administered at the same time than the adenylate cyclase preparation or at different times. FHA preparations are advantageously obtained using the method, for example, of Sato et al in Infect. Immun, 1983, 41, 313-320 or Imaizumi et al, Journal of Microbiol. Methods, 2, 334-347 (1984).

The adenylate cyclase preparations may also be associated with purified AC preparations disclosed in said FR patent application 8615963 of November 17, 1986, and optionally with FHA.

Advantageously, the vaccine compositions of the invention prevent the infection caused by Bordetella such as B.pertussis, B.parapertussis, B. bronchiseptica and B.avium.

According to another embodiment bacterial whole cell vaccines are elaborated from B.parapertussis. Such vaccines have a protective effect both against B.parapertussis and B.pertussis.

Other characteristics and advantages of the invention will be given in the following example, with reference to the figures, wherein :
- figure 1 represents the kinetics of pulmonary infection with B.parapertussis clones,
- figure 2 represents immunoblots of AC containing extracts from B. pertussis with polyclonal anti B. pertussis AC antibodies or serum antibodies from B. parapertussis intranasally infected mice.

The materials and methods used in the example are the followings :
a - Bacterial strains and culture conditions :
   B.pertussis 18323 (ATCC 9797) and B.parapertussis (CIP63-2) were plated on Bordet Gengou agar medium supplemented with horse defibrinated blood (15% final) (BG), for selecting hemolytic (hly) or non hemolytic clones, which were subcultured in Stainer Scholte broth.
b - Assays for AC and Ptx :
   Methods for detecting AC and Ptx, either from enzymatic or biological activities, or through Western blot analysis have been described by White A.A. et al Methods Enzymol., 38C, 41-46, 1974, such as modified by Hanoune et al, J. Biol. Chem., 252, 2039-2046, 1977.
c - Infection of mice :
   Three weeks old, specified pathogen-free, Swiss OF1 female mice, weighing 16 g + 1 were purchased from the ferme expérimentale de Rennemoulin (Institut Pasteur, 78 Villepreux, France) and maintained by groups of 5 in cages topped with filter caps (Techniplast, UAR, France), on sterile litter, and fed with gamma-ray sterilized food (granulés RO3, UAR, France) and autoclaved drinking water. The mice were infected at 4 weeks of age, after short ether anesthesia, by intranasal injection of standardized bacterial inocula (in a volume of 50 microlitres) from the B.pertussis strain Kendrick 18323 (type strain ATCC 9797), or the B.parapertussis CIP 63-2.
d - Histopathology :
   Lungs from infected mice were collected at selected timed fixed in 10% formaldehyde, embedded in paraffin and serialy sectionned before staining with hematoxilin and eosin or Giemsa, for light microscopy examination.

### Results :

### - KINETICS OF PULMONARY INFECTION WITH P.PARAPERTUSSIS

Two groups of mice were infected intranasally with inocula prepared from either an hly⁺ clone selected on BG (63-2H) or said hly⁺ clone, deposited under N° I 749 recovered from lungs homogenate of mice at day 3 post challenge (63-2S).

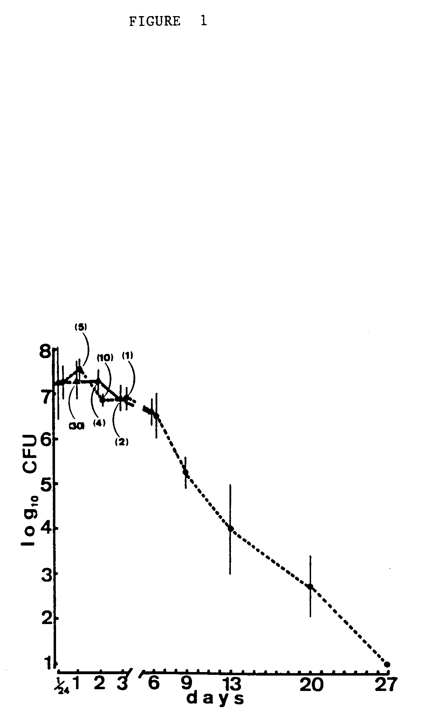

The dose-effects of the intranasal infection are reported on figure 1 which gives in function of days the variations of log₁₀ CFU/lungs (colony forming units) with 63-2H (curve •...•), and 63-2S (curve 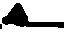 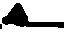 ). The vertical bars are arythmetic means of 5 mice ⁺ SEM.

As shown in figure 1, both inocula had similar virulence (in terms of bacterial growth/survival rate), whereas the clone 63-2S was significantly more pathogenic than the clone 63-2H. The intranasal injection of log₁₀ CFU corresponded to the LD50% in the case of 63-2S and only to the LD25% in the case of 63-2H.

### - HISTOPATHOLOGICAL FINDINGS

The lungs of mice dead at 24 h past challenge exhibited the typical AEHA observed with an hly⁺ AC⁺ clone derived from B.pertussis 18323, as reported by Brézin C. et al dans FEMS Microbiol. Lett. 42, 75-80, 1987.

### - AC ACTIVITIES

Dosages of AC in culture supernatants of 63-2S and 63-2H corresponding to standardized bacterial suspensions at 10 CFU/ml, showed that AC enzymatic activities were of 20-25 units and 4-7 units respectively.

### - CROSS-IMMUNITY BETWEEN B.PERTUSSIS AND B.PARAPERTUSSIS

Immune response of B.parapertussis infected mice to AC:
Western blot analysis of urea extracts of B.parapertussis with specific polyclonal anti-AC antibodies was carried out.

The urea extracts were obtained as follows :
Bacterial bottoms were homogeneized in buffer A (Tris HCl 25mM, MgCl₂ 6mM, NP40 0,1%, pH8), (1/3; w/w) and the same amount of urea 8 M in buffer A was added. The medium was stirred 40 min. at 30°C. A dialyzis in buffer A was performed, followed by a centrifugation (5000g) 30 min. at 6°C. The supernatant is recovered and constitutes the urea extract.

The polyclonal anti-AC are obtained by immunizing animals with purified AC preparations obtained from B.pertussis and recovering the antibodies raised against the preparations from the immunserums.

Preferred AC preparations used for raising the antibodies consist in the purified AC preparations disclosed in FR patent application 86/15963 filed on November 17, 1986, in the name of the applicant, relating to "Purified AC preparation, a method for obtaining the same and their biological applications".

Such preparations are characterized in that they possess a high degree of purity and are virtually totally devoid of contaminating bacterial substances, in particular pertussis toxin , lipopolysaccharide (or LPS) and filamentous hemagglutinin (or FHA). The AC is thus available in a homogeneous form, sedimenting with a S coefficient equal to 3.6 in a sucrose density gradient, which exists in two structurally related molecular forms of 45 and 43 kDa, respectively.

According to another aspect the preparations of AC are characterized in that they possess an activity which may attain and even exceed 1600 micromoles of cAMP min⁻¹. mg⁻¹.

Said purified AC preparations are obtainable by placing into contact with calmodulin a previously concentrated supernatant of bacterial cultures expressing adenylate cyclase or an extract of these bacteria.

The polyclonal antibodies are produced, for example, by immunizing animals such as the rabbit or the mouse by means of the AC preparations in question in which the AC is free or in the form of a complex with eucaryotic proteins, in particular with calmodulin, and by recovery of the antisera containing the antibodies and then of the antibodies themselves by standard methods.

The monoclonal antibodies are obtainable by fusion of a mouse myeloma with spleen lymphocytes derived from mice immunized with purified adenyl cyclase.

Monoclonal antibodies (8-25) are particlarly used. They immunoprecipitate a triplet of 50,45 and 43 kDa as well as a protein of molecular weight higher than 100 kDa in crude extracts and preparations of the enzyme purified from bacterial cells.

The hybridoma strain producing the monoclonal antibodies was deposited with the National Collection of Cultures of Microorganisms under the N° I-610 on the 9th of October 1986.

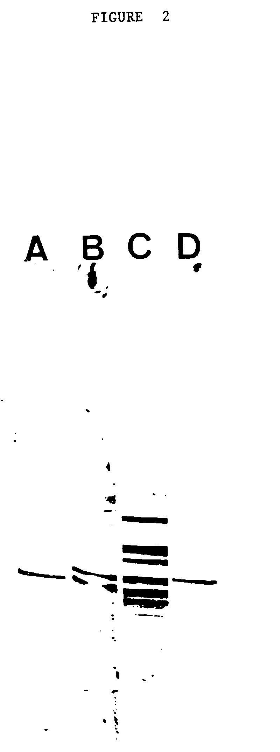

The results are given on figure 2 wherein lanes A to D respectively correspond to :
A. B. pertussis purified AC revealed with anti B. pertussis AC antibodies,
B. B. pertussis urea extract revealed with polyclonal anti-AC antibodies from B. parapertussis infected mice,
C. B. pertussis urea extract, revealed with serum from B. parapertussis infected mice,
D. B. pertussis purified AC revealed with serum antibodies from B. parapertussis infected mice.

Said Western blot analysis revealed the presence of a Mr 43-45 kDa antigen which cross reacted with the B.pertussis AC (figure 2).

Moreover, comparative immunoblotting of the purified B.pertussis AC with serum from B.parapertussis convalescing mice, collected at day 27 post in intranasal challenge (figure 1), revealed the presence of anti-B.pertussis AC antibodies.

### - PROTECTIVE EFFECTS OF ANTI-B.PERTUSSIS AC ANTIBODIES AGAINST THE RESPIRATORY LETHAL INFECTION OF THE MOUSE WITH B.PARAPERTUSSIS

a) B.pararertussis inoculum was preincubated with polyclonal or monoclonal anti-B.pertussis AC antibodies. The results are given in table 1 below :

**TABLE 1**

| Antibodies | Normal serum | Polyclonal anti-AC | | | Monoclonal anti-AC | | |
|---|---|---|---|---|---|---|---|
| log₁₀ dilution | -1 | -1 | -2 | -3 | -1 | -2 | -3 |
| N°Survivors/Total | 2/12 | 8/12 | 6/12 | 5/12 | 11/12 | 9/12 | 7/12 |

As shown in the table, said B.parapertussis inoculum incubated with polyclonal or monoclonal anti-B.pertussis AC antibodies inhibited the lethal challenge in a dose dependent fashion.

The cross-immunogenicity between B.parapertussis and B.pertussis was assessed by cross-vaccination experiments, as shown in table 2.

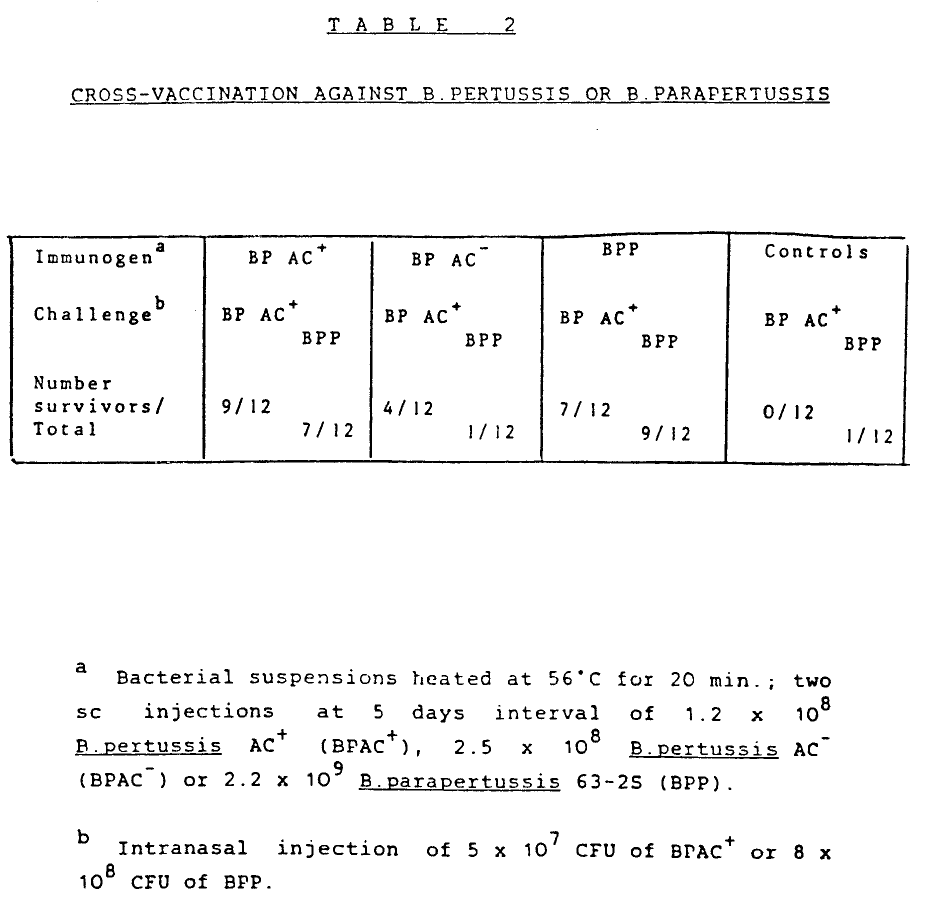

## Claims

1. Immunogenic composition characterised in that it contains an adenylate cyclase (AC) preparation, obtainable from B. parapertussis, said adenylate cyclase preparation comprising an active adenylate cyclase having a molecular weight (Mr) of about 43 ± 4.3 - 45 ± 4.5 kDa as determined with respect to ovalbumin as standard, specifically recognized by specific polyclonal and monoclonal anti-B. pertussis AC antibodies, and capable of antigenically cross-reacting with the B. pertussis AC, and/or an inactive protein having a molecular weight (Mr) of about 43 ± 4.3 kDa, specifically recognized by polyclonal anti-AC antibodies raised against purified AC preparation and devoid of adenylate cyclase calmodulin-activable activity and of affinity for calmodulin.

2. Immunogenic composition according to claim 1, characterised in that it is free of Ptx.

3. Immunogenic composition according to claim 1 characterised in that it comprises the inactive protein, optionally in combination with the active protein.

4. Immunogenic composition according to any one of claims 1-3 characterised in that it is protective against Bordetella infections in humans and animals.

5. Immunogenic composition according to any one of claims 1-4 characterised in that it further comprises FHA and/or AC purified from B. pertussis.

6. Immunogenic composition according to any one of claims 1-5 characterised in that it is a molecular vaccine.

7. Use of immunogenic composition according to any one of claims 1-6 for the preparation of a medicament to protect against infections by B. pertussis, B. parapertussis, B. bronchiseptica and B. avium.

8. Polyclonal or monoclonal antibodies capable of specifically recognising the AC preparation of claims 1-3.

9. Antibodies according to claim 8 characterised in that they are purified.
